# EUROPÄISCHE PATENTSCHRIFT

(11) **EP 3 891 117 B1**
(45) Veröffentlichungstag und Bekanntmachung des Hinweises auf die Patenterteilung: **03.01.2024**
(21) Anmeldenummer: 19809495.5
(22) Anmeldetag: 02.12.2019
(51) Int. Cl.: C07C 37/50, C07C 39/04

(54) **VERFAHREN ZUR HERSTELLUNG EINER HYDROXYVERBINDUNG DURCH DECARBOXYLIERUNG IN ANWESENHEIT EINER BRÖNSTED-BASE**
METHOD FOR PRODUCTION OF A HYDROXY COMPOUND BY MEANS OF DECARBOXYLATION IN THE PRESENCE OF A BRÖNSTED BASE
PROCÉDÉ DE PRODUCTION D'UN COMPOSÉ HYDROXY PAR DECARBOXYLATION EN PRÉSENCE D'UNE BASE DE BRÖNSTED

(30) Priorität: 07.12.2018 EP 18211034
(43) Veröffentlichungstag der Anmeldung: 13.10.2021
(73) Patentinhaber: Covestro Intellectual Property GmbH & Co. KG, 51373 Leverkusen (DE)
(72) Erfinder: LANGANKE, Jens, 53894 Mechernich (DE); SLUYTS, Erik, 2930 Brasschaat (BE); HEIJL, Jan, 9160 Lokeren (BE); MEINE, Niklas, 40223 Düsseldorf (DE)
(74) Vertreter: Levpat
(86) Internationale Anmeldenummer: PCT/EP2019/083211
(87) Internationale Veröffentlichungsnummer: WO 2020/114925

(56) Entgegenhaltungen:
- EP-A1- 1 277 723
- EP-A1- 2 586 767
- WO-A1-2013/035103
- GB-A- 818 434
- JP-A- 2016 023 136
- US-A- 2 488 472
- US-A1- 2004 143 867

## Beschreibung

Die vorliegende Erfindung betrifft ein Verfahren zur Herstellung einer speziellen Hydroxyverbindung durch Decarboxylierung einer speziellen Carbonsäureverbindung oder eines Salzes dieser Carbonsäureverbindung in Anwesenheit einer Brönsted-Base, ein Verfahren zur Herstellung eines Diarylcarbonats oder eines Bisphenols, ein Verfahren zur Herstellung eines Polycarbonats und eine Verwendung einer Brönsted-Base bei der Reaktion der Decarboxylierung einer speziellen Carbonsäureverbindung oder eines Salzes dieser Carbonsäureverbindung.

Phenole mit unterschiedlichen Substitutionsmustern am Aromaten stellen die Ausgangsverbindungen für unterschiedliche Monomere und damit auch für die daraus resultierenden Polymere dar. Die Herstellung solcher Phenole aus nachwachsenden Rohstoffen stellt eine große Herausforderung dar. Eine Möglichkeit zur Herstellung biobasierten Phenols bietet die direkte Fermentation von Zuckern, wie beispielsweise in der WO 2014/076113 A1 beschrieben. Phenol ist jedoch für den dort beschriebenen Mikroorganismus toxisch und auch die Abtrennung aus der wässrigen Fermentationsbrühe ist aufwendig. Hydroxybenzoesäuren wie beispielsweise 4-Hydroxybenzoesäure, 2-Hydroxybenzoesäure und 3-Hydroxybenzoesäure können ebenfalls fermentativ aus Zuckern hergestellt werden. Da sie für die eingesetzten Mikroorganismen in der Regel weniger toxisch sind, können im Vergleich zu Phenol normalerweise höhere Ausbeuten erzielt werden. Hydroxybenzoesäuren können kristallisiert und aus der Fermentationsbrühe abgetrennt werden. Eine anschließende Decarboxylierung von 4-Hydroxybenzoesäure zu Phenol wurde auch bereits beschrieben. Die JP 2016-23136 A beschreibt die Reaktion unter Verwendung eines heterogenen Katalysators in Wasser als Lösungsmittel. In A.S. Lisitsyn / Applied Catalysis A: General 332 ; 2007 (166-170) wird die Decarboxylierung in Diphenylether unter Verwendung eines Kupferkatalysators beschrieben. L. J. Gooßen et al. beschreiben in ChemCatChem 2010, 2, 430-442 die Decarboxylierung mittels Silber- oder Kupferkatalysator in NMP als Lösungsmittel. Auch in Dalton Transactions (24), 4683-4688; 2009 wird die Decarboxylierung in Toluol beschrieben.

Die beschriebenen Methoden weisen jedoch alle Verbesserungspotential in Bezug auf die Reaktionsbedingungen, Ausbeute und Selektivität auf.

Der vorliegenden Erfindung lag daher die Aufgabe zugrunde, ein Verfahren zur Herstellung von speziellen Hydroxyverbindungen der Formel (I) mittels Decarboxylierung einer Carbonsäureverbindung der Formel (II) oder eines entsprechenden Salzes dieser Carbonsäureverbindung der Formel (II) bereitzustellen, welches mindestens einen Nachteil des Stands der Technik verbessert. Insbesondere lag der vorliegenden Erfindung die Aufgabe zu Grunde, ein Verfahren bereitzustellen, welches die Hydroxyverbindung der Formel (I) in hoher Ausbeute liefert. Diese hohe Ausbeute sollte bevorzugt in entsprechend kurzer Zeit erzielt werden. Besonders bevorzugt sollte die Ausbeute höher sein als in im Stand der Technik beschriebenen Verfahren. Ebenso bevorzugt sollte die Reaktionszeit mit entsprechend hoher Ausbeute kürzer sein als in im Stand der Technik beschriebenen Verfahren.

Mindestens eine, bevorzugt alle der oben genannten Aufgaben wurden durch die vorliegende Erfindung gelöst. Überraschend wurde gefunden, dass die Decarboxylierung einer Carbonsäureverbindung der Formel (II) oder eines entsprechenden Salzes dieser Carbonsäureverbindung der Formel (II) besonders effektiv abläuft, wenn bei der Reaktion der Decarboxylierung mindestens eine Brönsted-Base anwesend ist. Bevorzugt ist die Ausbeute an der gewünschten Hydroxyverbindung der Formel (I) sogar höher als unter den im Stand der Technik beschriebenen Bedingungen. Dies wird ebenso bevorzugt in kürzerer Zeit als unter den im Stand der Technik beschriebenen Bedingungen erzielt. Zudem wurde eine hohe Selektivität beobachtet. Die hohe Selektivität in Kombination mit hoher Ausbeute bietet insbesondere den Vorteil, dass ein Reaktionsprodukt erhalten wird, aus dem das Edukt (eine Carbonsäureverbindung der Formel (II) oder ein entsprechendes Salz dieser Carbonsäureverbindung der Formel (In) nicht aufwendig abgetrennt werden muss, sondern für weitere Reaktionen eingesetzt werden kann.

Erfindungsgemäß wird daher ein Verfahren zur Herstellung einer Hydroxyverbindung der Formel (I) bereitgestellt 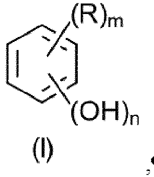 in der
R für eine lineare oder verzweigte Alkylgruppe mit 1 bis 6 Kohlenstoffatomen steht,
n 1 oder 2 ist und
m 0, 1, 2 oder 3 ist,
durch Decarboxylierung einer Carbonsäureverbindung der Formel (II) oder eines entsprechenden Salzes dieser Carbonsäureverbindung der Formel (II) 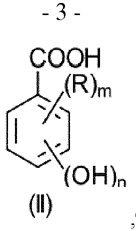 in der R, n und m die oben genannten Bedeutungen haben,
gegebenenfalls unter Verwendung mindestens eines heterogenen Katalysators,
wobei das erfindungsgemäße Verfahren dadurch gekennzeichnet ist, dass bei der Reaktion der Decarboxylierung mindestens eine Brönsted-Base anwesend ist,
wobei die Brönsted-Base kein Ammoniak, primäres Amin, sekundäres Amin oder ein Gemisch aus zweien oder mehreren dieser stickstoffhaltigen Brönsted-Basen ist und
wobei die Reaktion der Decarboxylierung in einem wässrigen Medium durchgeführt wird.

Der Begriff der "Brönsted-Base" ist dem Fachmann bekannt. Insbesondere versteht er darunter eine Verbindung, die ein Proton aufnehmen kann und somit als Protonenakzeptor wirkt. Bevorzugt ist das erfindungsgemäße Verfahren dadurch gekennzeichnet, dass die mindestens eine Brönsted-Base ausgewählt wird aus der Gruppe, bestehend aus Natriumhydroxid, Kaliumhydroxid, Natriumphenolat, Natriumacetat, Natriumphosphat und beliebigen Mischungen daraus. Besonders bevorzugt wird die mindestens eine Brönsted-Base ausgewählt aus der Gruppe, bestehend aus Natriumhydroxid, Kaliumhydroxid, Natriumphenolat und beliebigen Mischungen daraus. Ganz besonders bevorzugt ist die mindestens eine Brönsted-Base Natriumhydroxid. Dies ist insbesondere vorteilhaft, da es die hergestellte Hydroxyverbindung der Formel (I) besonders geeignet macht für die Verwendung zur Herstellung eines Bisphenols, eines Diarylcarbonats und/oder eines Polycarbonats, da zur Synthese dieser Substanzen häufig ebenfalls Natriumhydroxid benötigt wird. Dies bedeutet, dass eventuelle Verunreinigungen der Hydroxyverbindungen der Formel (I) durch die Brönsted-Base ihre weitere Umsetzung zum gewünschten Zielprodukt nicht wesentlich stören.

Für das erfindungsgemäße Verfahren ist eine stickstoffhaltige Brönstedt-Base wie Ammoniak, ein primäres Amin, beispielsweise Monomethylamin oder Monoethylamin, oder ein sekundäres Amin, beispielsweise Dimethylamin oder Diethylamin, oder ein Gemisch aus zweien oder mehreren dieser stickstoffhaltigen Brönstedt-Basen zu vermeiden.

Eine Brönstedt-Base dieser Art ist unvorteilhaft, da Verunreinigungen durch eine stickstoffhaltige Brönstedt-Base dieser Art die hergestellte Hydroxyverbindung der Formel (I) ungeeignet macht für die Verwendung zur Herstellung eines Bisphenols, eines Diarylcarbonats und/oder eines Polycarbonats. Grund dafür ist, dass bei der Verwendung der Hydroxyverbindung der Formel (I) zur Herstellung von Polycarbonaten eine solche stickstoffhaltige Brönsted-Base Nebenreaktionen verursachen kann, bei denen Carbamatverbindungen entstehen. Solche Carbamatverbindungen sind in Polycarbonaten unerwünscht, weil damit Stickstoff in die Polymerkette eingebaut wird, der die Eigenschaften des Polycarbonats verschlechtert, wie bereits aus der DE10300598A1 bekannt ist. Dies bedeutet, dass eventuelle Verunreinigungen der Hydroxyverbindungen der Formel (I) durch eine Brönsted-Base dieser Art bei der weiteren Umsetzung der Hydroxyverbindungen der Formel (I) zum gewünschten Zielprodukt empfindlich stören können.

Das erfindungsgemäße Verfahren schließt daher die Verwendung von Ammoniak, primären Aminen und sekundären Aminen ganz aus.

Die Verwendung stickstoffhaltiger Brönstedt-Basen, die tertiäre Amine sind, beispielsweise Triethylamin, Tributylamin oder N-Ethylpiperidin, sind jedoch nicht vom erfindungsgemäßen Verfahren ausgeschlossen. Bei Verwendung von tertiären Aminen kommt es nicht zur Bildung von Carbamaten, wenn diese tertiären Amine noch als Verunreinigungen der hergestellten Hydroxyverbindung der Formel (I) vorliegen sollten.

Experimentell wurde von den Erfindern beobachtet, dass unter Zugabe einer Brönsted-Base bei der Reaktion der Decarboxylierung eine höhere Ausbeute des gewünschten Zielproduktes erhalten wurde bei ansonsten gleichen Reaktionsbedingungen aber ohne Base. Zudem konnte durch verfrühten Abbruch der Reaktion gesehen werden, dass durch die Zugabe der Brönsted-Base zu einem deutlich früheren Zeitpunkt dieselbe Ausbeute erhalten wurde wie ohne die Zugabe der Brönsted-Base. Diese Beobachtungen waren insbesondere überraschend, da in der Literatur häufig die Decarboxylierung mittels Säure katalysiert wird. Dabei ist es bevorzugt, wenn die mindestens eine Brönsted-Base in einer Konzentration von 0,0001 mol/L bis 20 mol/L, besonders bevorzugt 0,001 mol/L bis 5 mol/L, besonders bevorzugt 0,001 mol/L bis 1 mol/L, besonders bevorzugt 0,01 mol/L bis 0,1 mol/L, besonders bevorzugt 0,001 mol/L bis 0,05 mol/L und ganz besonders bevorzugt 0,009 mol/L bis 0,02 mol/L, bezogen auf die gesamte Reaktionsmischung der Decarboxylierung, anwesend ist. Es hat sich herausgestellt, dass in diesen Konzentrationen die Brönsted-Base die gewünschte katalytische Aktivität in Bezug auf die Reaktion der Decarboxylierung zeigt und gleichzeitig die Konzentration der Brönsted-Base so gering ist, dass negative Einflüsse wie beispielsweise Korrosion der für die Reaktion verwendeten Vorrichtungen minimal gehalten werden kann.

Das erfindungsgemäße Verfahren wird in einem "wässrigen Medium" in Lösung durchgeführt. J Z Die Begriffe "lösen" und "in Lösung" sind erfindungsgemäß gemäß dem Wissen des Fachmanns zu verstehen. Bevorzugt bedeutet "lösen" und "in Lösung", wenn bei Filtration einer Flüssigkeit, in der eine Substanz gelöst ist, mit gängigen Filtermethoden kein Feststoff abgetrennt werden kann. Erfindungsgemäß ist es daher bevorzugt, dass die Carbonsäureverbindung der Formel (II) oder das entsprechende Salz der Carbonsäureverbindung der Formel (II) in einem Lösungsmittel gelöst wird, bevor die Reaktion der Decarboxylierung durchgeführt wird. Der Vorgang des Lösens kann dabei auf einem dem Fachmann bekannte Weise auch unter Erwärmung stattfinden. Der Begriff des Lösungsmittels ist dem Fachmann bekannt. Es hat sich als erfindungsgemäß vorteilhaft herausgestellt, dass die Reaktion der Decarboxylierung in einem wässrigen Medium durchgeführt wird. Dies bedeutet, dass zumindest ein Teil des verwendeten Lösungsmittels Wasser ist. Bevorzugt wird unter dem Begriff "wässriges Medium" verstanden, dass mindestens 10 Vol.-% des eingesetzten Lösungsmittels Wasser sind, bevorzugt mindestens 50 Vol.-%, besonders bevorzugt mindestens 80 Vol.-% und weiterhin bevorzugt mindestens 90 Vol.-% Besonders bevorzugt besteht das wässrige Medium aus Wasser. Hierbei ist jedoch von einer gängigen Wasserqualität unter Anwesenheit entsprechender Ionen auszugehen.

Diese Ausführungsform bietet insbesondere den Vorteil, dass wenig bis gar keine organischen Lösungsmittel eingesetzt werden. Diese müssen im Anschluss nicht in einem zusätzlichen Schritt abgetrennt werden. Insbesondere werden durch den Verzicht auf ein organisches Lösungsmittels auch keine weiteren unerwünschten organischen Verunreinigungen ins System eingeführt.

Wie bereits oben beschrieben, konnte durch die Zugabe der mindestens einen Brönsted-Base die Reaktionszeit im Vergleich zu einer ansonsten gleichen Reaktion ohne Base verkürzt werden. Daher ist es erfindungsgemäß bevorzugt, dass die Reaktionszeit der Reaktion der Decarboxylierung länger als 5 min und kürzer als 48 h, besonders bevorzugt länger als 25 min und kürzer als 24 h, ebenso bevorzugt länger als 40 min und kürzer als 12 h und ganz besonders bevorzugt länger als 1 h und kürzer als 5 h beträgt. Dem Fachmann ist bekannt, dass bei höheren Konzentrationen des Katalysators, in diesem Fall der Brönsted-Base, kürzere Reaktionszeiten für eine hohe Ausbeute ausreichend sein können.

Beim erfindungsgemäßen Verfahren kann es sich um ein batch, semi-batch oder kontinuierliches Verfahren handeln.

Bevorzugt wird das erfindungsgemäße Verfahren bei einer Temperatur von 20 bis 400 °C, besonders bevorzugt bei 100 bis 350 °C, besonders bevorzugt bei 150 bis 300 °C und ganz besonders bevorzugt von 160 bis 250 °C durchgeführt.

Bevorzugt dient das erfindungsgemäße Verfahren zur Herstellung einer Hydroxyverbindung der oben gezeigten Formel (I), in der R für eine tert-Butyl-, Propyl- oder Methylgruppe steht, n 1 oder 2, bevorzugt 1 ist und m 0, 1, 2 oder 3 ist. Besonders bevorzugt dient das erfindungsgemäße Verfahren der Herstellung von 4-Propylphenol, ortho-, para- oder metha-Methylphenol (Cresole), 2,4-Dimethylphenol, 2,5-Dimethylphenol, 4-tert-Butyl-phenol oder Phenol. Ganz besonders bevorzugt ist das erfindungsgemäße Verfahren dadurch gekennzeichnet, dass die Hydroxyverbindung der Formel (I) Phenol ist.

Erfindungsgemäß werden die Carbonsäureverbindung der Formel (II) oder das Salz der Carbonsäureverbindung der Formel (II) gelegentlich auch als Carbonsäureverbindung der Formel (II) zusammengefasst. Gemeint sind aber immer die freie Säure und/oder das Salz, soweit nicht anders angegeben. Erfindungsgemäß können auch Mischungen unterschiedlicher Carbonsäureverbindungen der Formel (II) oder unterschiedlicher Salze der Carbonsäureverbindungen der Formel (II) oder auch Mischungen mindestens einer Carbonsäureverbindung der Formel (II) mit mindestens einem Salz der Carbonsäureverbindung der Formel (II) eingesetzt werden.

Im erfindungsgemäßen Verfahren ist es bevorzugt, dass das Kation des Salzes der Carbonsäureverbindung der Formel (II) ausgewählt wird aus der Gruppe bestehend aus Alkalimetallkationen, Erdalkalimetallkationen, Ammonium, Phosphonium, Kationen von Mangan, Eisen, Kobalt, Nickel, Kupfer, Zink, Molybdän, Cadmium und beliebigen Mischungen davon. Besonders bevorzugt ist das Kation des Salzes der Carbonsäureverbindung der Formel (II) ausgewählt aus der Gruppe bestehend aus Alkalimetallkationen, Erdalkalimetallkationen und Mischungen davon.

Des Weiteren ist es erfindungsgemäß bevorzugt, dass die Carbonsäureverbindung der Formel (II) oder das entsprechende Salz der Carbonsäureverbindung der Formel (II) ausgewählt wird aus der Gruppe, bestehend aus 2-Hydroxybenzoesäure, 4-Hydroxybenzoesäure und den entsprechenden Salzen. Ganz besonders bevorzugt handelt es sich um 4-Hydroxybenzoesäure oder das entsprechende Salz.

Wenn ein Katalysator im erfindungsgemäßen Verfahren verwendet wird, kommen grundsätzlich alle heterogenen Katalysatoren in Frage, welche bei einer Decarboxylierungsreaktion aktiv sind. Diese sind dem Fachmann bekannt. Bevorzugt wird der im erfindungsgemäßen Verfahren eingesetzte heterogene Katalysator ausgewählt aus der Gruppe, bestehend aus Al₂O₃, H₃PO₄ geträgert auf Al₂O₃, PtClₓ geträgert auf Al₂O₃, Cu/Al/Ga-MOFs, Pt-Al-MOFs, Palladium geträgert auf Aktivkohle, Platin geträgert auf Aktivkohle, Zeolithe, wie beispielsweise ZSM-5, HZSM-5, Fe₂O₃ geträgert auf MCM-41 (Mobil Composition of Matter No. 41), Fe₂O₃ geträgert auf Al-MCM-41, Pt geträgert auf SAPO-34 (Silicoaluminophosphat), Pt geträgert auf SAPO-11, Pt Hydrotalcite, Pt geträgert auf SiO₂ und beliebigen Mischungen davon. Besonders bevorzugt ist das erfindungsgemäße Verfahren dadurch gekennzeichnet, dass der mindestens eine gegebenenfalls verwendete heterogene Katalysator ein Zeolith ist. Ganz besonders bevorzugt ist das erfindungsgemäße Verfahren dadurch gekennzeichnet, dass das Zeolith eine Faujasit-Struktur aufweist.

Dem Fachmann sind Zeolithe und insbesondere Zeolithe mit Faujasit-Struktur bekannt. Die Kristallstruktur von Faujasit ist identisch mit der des synthetischen Zeolith Y. Das Grundelement des Faujasitgerüsts sind Sodalithkäfige, die über hexagonale Prismen miteinander verbunden sind. Ganz besonders bevorzugt ist der erfindungsgemäß eingesetzte Katalysator vom Zeolith Y Typ.

Erfindungsgemäß kann das Verfahren mit und ohne Katalysator ausgeführt werden. Dabei ist es bevorzugt, dass das Verfahren entweder komplett ohne Katalysator oder mit einem der oben genannten bevorzugten Katalysatoren durchgeführt wird. Dabei ist unter einer Reaktion "ohne Katalysator" die Anwesenheit der mindestens einen Brönsted-Base nicht ausgeschlossen, sondern erfindungsgemäß gefordert.

In einem Aspekt der Erfindung ist es des Weiteren bevorzugt, dass die Carbonsäureverbindung der Formel (II) oder das entsprechende Salz der Carbonsäureverbindung der Formel (II) durch Fermentation oder aus Zuckern, Lignocellulose, lignocellulose-haltigen Materialien, Furanen und/oder Lignin erhalten wurde. Damit ist die Carbonsäureverbindung der Formel (II) oder das entsprechende Salz der Carbonsäureverbindung der Formel (II) bevorzugt biobasiert. Im Sinne der vorliegenden Erfindung wird unter dem Ausdruck "biobasiert" verstanden, dass die betreffende chemische Verbindung zum Anmeldezeitpunkt durch einen erneuerbaren und/oder nachwachsenden Rohstoff zugänglich, erhältlich und/oder bevorzugt ein solcher erneuerbarer und/oder nachwachsender Rohstoff ist. Unter einem erneuerbaren und/oder nachwachsenden Rohstoff wird bevorzugt ein Rohstoff verstanden, welcher durch natürliche Prozesse mit einer Geschwindigkeit regeneriert wird, die mit ihrer Abbaurate vergleichbar ist (Siehe CEN/TS 16295:2012). Der Ausdruck dient insbesondere der Abgrenzung zu Rohstoffen aus fossilen Rohstoffen, erfindungsgemäß auch als petrobasiert bezeichnet. Ob ein Rohstoff biobasiert ist oder petrobasiert, kann durch die Messung von Kohlenstoffisotopen in dem Rohstoff festgestellt werden, da die relativen Mengen des Kohlenstoffisotops C14 geringer sind in fossilen Rohstoffen.

Dies kann beispielsweise gemäß der ASTM D6866-18 (2018) oder der ISO16620-1 bis -5 (2015) oder der DIN SPEC 91236 2011-07 erfolgen.

Erfindungsgemäß wird der Begriff "petrobasiert" bevorzugt für solche Verbindungen verwendet, welche einen C14 Isotopengehalt von unter 0,3 × 10⁻¹², besonders bevorzugt von 0,2 × 10⁻¹² und ganz besonders bevorzugt von 0,1 × 10⁻¹² aufweist.

Dem Fachmann ist bekannt, wie er Carbonsäureverbindung der Formel (II) oder das entsprechende Salz der Carbonsäureverbindung der Formel (II) durch Fermentation oder aus Zuckern, Lignocellulose, lignocellulose-haltigen Materialien, Furanen und/oder Lignin erhalten kann. Dies ist beispielsweise in der WO 2015174446, WO 2015156271, US20040143867, Appl. Environ Microbiol 84 2018 :e02587-17, WO2016114668, Biomass and Bioenergy 93:209-216 October 2016, Biotechnol Bioeng. 2016 Jul;113(7):1493-503, ACS Catal., 2016, 6 (9), pp 6141-6145 oder Biotechnol. Bioeng., 113: 1493-1503, Appl Microbiol Biotechnol. 2018 Oct;102(20):8685-8705, Microbiology. 1994 Apr;140 ( Pt 4):897-904, Journal of Biotechnology 132 (2007) 49-56, WO2000018942, US 6030819, EP2698435, Bioprocess Biosyst Eng (2017) 40: 1283, US2996540, US9206449, Nature 2014, 515, 249-252, Biomass and Bioenergy 93 (2016) 209-216, 3 Biotech. 2015 Oct; 5(5): 647-651, Appl Environ Microbiol. 2018 Mar 15; 84(6): e02587-17, US3360553A beschrieben.

In diesem Aspekt der Erfindung ist es besonders vorteilhaft, dass durch den Einsatz einer biobasierten Carbonsäureverbindung der Formel (II) oder eines entsprechenden Salzes der Carbonsäureverbindung der Formel (II) eine biobasierte Hydroxyverbindung der Formel (I) erhalten wird. Diese wiederum kann zur Herstellung von weiteren biobasierten Verbindungen, beispielsweise Diarylcarbonaten, Bisphenolen oder Polycarbonaten genutzt werden wodurch letzendlich biobasierte Polymere zugänglich und auf einem effizienten und kostengünstigen Weg hergestellt werden.

In einem weiteren Aspekt der vorliegenden Erfindung wird ein Verfahren zur Herstellung eines Diarylcarbonats oder eines Bisphenols bereitgestellt, welches dadurch gekennzeichnet ist, dass es die folgenden Schritte umfasst:
(i) Durchführung des Verfahrens in sämtlichen Ausgestaltungen und Bevorzugungen, um eine Hydroxyverbindung der Formel (I) zu erhalten und
(ii) Reaktion der Hydroxyverbindung der Formel (I) aus Schritt (i) mit mindestens einem Keton, um ein Bisphenol zu erhalten oder Reaktion der Hydroxyverbindung der Formel (I) aus Schritt (i) mit Phosgen, um ein Diarylcarbonat zu erhalten.

Es hat sich als besonders vorteilhaft herausgestellt, dass durch die hohe Selektivität im Verfahrensschritt (i), wie bereits oben beschrieben, eine besonders reine Hydroxyverbindung der Formel (I) erhalten wird. Dies kann ohne aufwendige Aufarbeitung dann direkt weiter verarbeitet werden. Bevorzugt erfolgt vorher gegebenenfalls eine Abtrennung des heterogenen Katalysators, falls vorhanden, und/oder des gegebenenfalls vorhandenen Lösungsmittels. Die Abtrennung des gegebenenfalls vorhandenen Katalysators ist dem Fachmann bekannt. Sie kann beispielsweise durch Filtration erzielt werden. Ebenso ist dem Fachmann die Abtrennung des gegebenenfalls vorhandenen Lösungsmittels bekannt. Diese kann beispielsweise durch Destillation, Absorption oder ähnliche Trennverfahren durchgeführt werden. In Abhängigkeit von der Konzentration der verwendeten Brönsted-Base kann diese gegebenenfalls auch zunächst noch neutralisiert werden oder auch im System verbleiben. Die Neutralisierung kann beispielsweise durch Zugabe von HCl erfolgen. Das gebildete NaCl kann entweder vor der Weiterverwendung durch dem Fachmann bekannte gängige Methode abgetrennt werden oder, ähnlich wie die Brönsted-Base, in der Mischung verbleiben, da es beim erfindungsgemäßen Verfahren zur Herstellung eines Diarylcarbonats oder eines Bisphenols in geringen Konzentrationen unerhebliche Auswirkungen auf den Reaktionsverlauf hat.

Erfindungsgemäß ist es in diesem Fall besonders bevorzugt, dass es sich bei der Carbonsäureverbindung der Formel (II) oder dem Salz der Carbonsäureverbindung der Formel (II) um 4-Hydroxybenzoesäure oder das entsprechende Salz handelt.

Verfahren zur Herstellung von Diarylcarbonaten oder Bisphenolen sind dem Fachmann bekannt. Diarylcarbonate können beispielsweise durch die Reaktion der Hydroxyverbindung der Formel (I) mit Phosgen auf bekannte Art und Weise hergestellt werden. Bisphenole können durch Reaktion der Hydroxyverbindung der Formel (I) mit einem Keton auf bekannte Art und Weise erhalten werden. Bei diesen Verfahren können die weiteren Reaktionspartner, wie beispielsweise die Ketone, ebenfalls biobasiert oder petrobasiert, bevorzugt biobasiert sein. Dadurch können Produkte mit unterschiedlichen Anteilen an biobasiertem Kohlenstoff gezielt erhalten werden.

Derzeit existieren unterschiedliche Kennzeichnungen, ab wann ein Produkt als "Biobasiert" bezeichnet werden darf (siehe unter anderem das Zertifizierungsprogramm "biobasierte Produkte nach ASTM D6866-18 (2018) oder der ISO16620-1 bis -5 (2015) oder der DIN SPEC 91236 2011-07 vom TÜVRheinland^{®}). Diese unterschiedlichen Kennzeichnungen setzen eine bestimmte Prozentzahl an biobasiertem Kohlenstoff im Produkt voraus. Das erfindungsgemäße Verfahren ermöglicht es, den Anteil an biobasiertem Kohlenstoff einfach einzustellen.

Bevorzugte Bisphenole, die mit dem erfindungsgemäßen Verfahren hergestellt werden können, sind solche der Formel (2a)

HO-Z-OH (2a),

in welcher
Z ein aromatischer Rest mit 6 bis 30 C-Atomen ist, der einen oder mehrere aromatische Kerne enthalten kann, substituiert sein kann und aliphatische oder cycloaliphatische Reste bzw. Alkylaryle oder Heteroatome als Brückenglieder enthalten kann.

Bevorzugt steht Z in Formel (2a) für einen Rest der Formel (3) 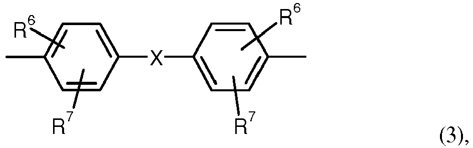 in der
R⁶ und R⁷unabhängig voneinander für H, C₁- bis C₁₈-Alkyl-, C₁- bis C₁₈-Alkoxy, Halogen wie Cl oder Br oder für jeweils gegebenenfalls substituiertes Aryl- oder Aralkyl, bevorzugt für H oder C₁- bis C₁₂-Alkyl, besonders bevorzugt für H oder C₁- bis Cs-Alkyl und ganz besonders bevorzugt für H oder Methyl stehen, und
X für eine Einfachbindung, -SO₂-, -CO-, -O-, -S-, C₁- bis C₆-Alkylen, C₂- bis Cs-Alkyliden oder C₅- bis C₆-Cycloalkyliden, welches mit C₁- bis C₆-Alkyl, vorzugsweise Methyl oder Ethyl, substituiert sein kann, ferner für C₆- bis C₁₂-Arylen, welches gegebenenfalls mit weiteren Heteroatome enthaltenden aromatischen Ringen kondensiert sein kann, steht.

Bevorzugt steht X für eine Einfachbindung, C₁- bis C₅-Alkylen, C₂- bis C₅-Alkyliden, C₅- bis C₆-Cycloalkyliden, -O-, -SO-, -CO-, -S-, -SO₂-
oder für einen Rest der Formel (3a)

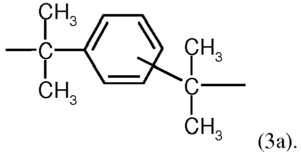

Beispiele für Bisphenole sind: Dihydroxybenzole, Dihydroxydiphenyle, Bis-(hydroxyphenyl)-alkane, Bis-(hydroxyphenyl)-cycloalkane, Bis-(hydroxyphenyl)-aryle, Bis-(hydroxyphenyl)-ether, Bis-(hydroxyphenyl)-ketone, Bis-(hydroxyphenyl)-sulfide, Bis-(hydroxyphenyl)-sulfone, Bis-(hydroxyphenyl)-sulfoxide, 1,1'-Bis-(hydroxyphenyl)-diisopropylbenzole sowie deren kernalkylierte und kernhalogenierte Verbindungen.

Bevorzugte Bisphenole sind 4,4'-Dihydroxydiphenyl, 2,2-Bis-(4-hydroxyphenyl)-1-phenylpropan, 1,1-Bis-(4-hydroxyphenyl)-phenylethan, 2,2-Bis-(4-hydroxyphenyl)propan, 2,4-Bis-(4-hydroxyphenyl)-2-methylbutan, 1,3-Bis-[2-(4-hydroxyphenyl)-2-propyl]benzol (Bisphenol M), 2,2-Bis-(3-methyl-4-hydroxyphenyl)-propan, Bis-(3,5-dimethyl-4-hydroxyphenyl)-methan, 2,2-Bis-(3,5-dimethyl-4-hydroxyphenyl)-propan, Bis-(3,5-dimethyl-4-hydroxyphenyl)-sulfon, 2,4-Bis-(3,5-dimethyl-4-hydroxyphenyl)-2-methylbutan, 1,3-Bis-[2-(3,5-dimethyl-4-hydroxyphenyl)-2-propyl]-benzol und 1,1-Bis-(4-hydroxyphenyl)-3,3,5-trimethylcyclohexan (Bisphenol TMC).

Besonders bevorzugte Bisphenole sind 4,4'-Dihydroxydiphenyl, 1,1-Bis-(4-hydroxyphenyl)-phenyl-ethan, 2,2-Bis-(4-hydroxyphenyl)-propan, 2,2-Bis(3,5-dimethyl-4-hydroxyphenyl)-propan, 1,1-Bis-(4-hydroxyphenyl)-cyclohexan und 1,1-Bis-(4-hydroxyphenyl)-3,3,5-trimethylcyclohexan (Bisphenol TMC).

Bevorzugte Diarylcarbonate, die mit dem erfindungsgemäßen Verfahren hergestellt werden können, sind die der Formel (2) 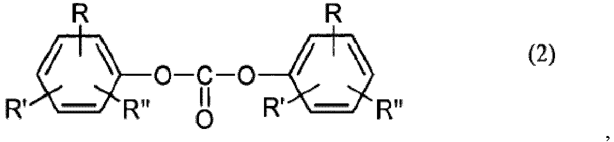 wobei R, R' und R" jeweils unabhängig voneinander gleich oder verschieden sein können und für Wasserstoff, gegebenenfalls verzweigtes C1-C34-Alkyl, C7-C34-Alkylaryl oder C6-C34-Aryl stehen, R weiterhin auch -COO-R‴ bedeuten kann, wobei R‴ für gegebenenfalls verzweigtes C1-C34-Alkyl, C7-C34-Alkylaryl oder C6-C34-Aryl steht. Solche Diarylcarbonate sind beispielsweise in EP-A 1 609 818 beschrieben. Bevorzugt sind Diphenylcarbonat, 4-tert-Butylphenyl-phenylcarbonat, Di-(4-tert-butylphenyl)-carbonat, Biphenyl-4-yl-phenyl-carbonat, Di-(biphenyl-4-yl)-carbonat, 4-(1 -Methyl-1 -phenylethyl)-phenyl-phenyl-carbonat und Di-[4-(1 -methyl-1-phenylethyl)-phenyl]-carbonat. Ganz besonders bevorzugt ist substituiertes oder unsubstituiertes, bevorzugt unsubstituiertes Diphenylcarbonat.

In einem weiteren Aspekt der vorliegenden Erfindung wird ein Verfahren zur Herstellung eines Polycarbonats unter Polymerisation mindestens eines Bisphenols und/oder mindestens eines Diarylcarbonats bereitgestellt, dadurch gekennzeichnet, dass entweder
(A) das mindestens eine Bisphenol gemäß dem oben beschriebenen erfindungsgemäßen Verfahren zur Herstellung eines Bisphenols in sämtlichen Ausgestaltungen und Bevorzugungen hergestellt wird und/oder das mindestens eine Diarylcarbonat gemäß dem oben beschriebenen erfindungsgemäßen Verfahren zur Herstellung eines Diarylcarbonats in sämtlichen Ausgestaltungen und Bevorzugungen hergestellt wurde oder
(B) dass eine Hydroxyverbindung der Formel (I) hergestellt wird nach dem oben beschriebenen Verfahren zur Herstellung einer Hydroxyverbindung der Formel (I) in sämtlichen Ausgestaltungen und Bevorzugungen und anschließend als Kettenabbrecher in der Polymerisation des Polycarbonats nach dem Phasengrenzflächenverfahren eingesetzt wird.

Solche Verfahren zur Herstellung von Polycarbonat mittels Polymersiation von Diarylcarbonaten und/oder Bisphenolen sind dem Fachmann bekannt. Beispielsweise können die Bisphenole und evtl. Verzweiger in wässriger alkalischer Lösung gelöst werden und mit einer gegebenenfalls in einem Lösemittel gelösten Carbonatquelle wie Phosgen in einem Zweiphasengemisch aus einer wässrigen alkalischen Lösung, einem organischen Lösemittel und einem Katalysator, bevorzugt einer Aminverbindung, zur Reaktion gebracht werden. Die Reaktionsführung kann auch mehrstufig erfolgen. Solche Verfahren zur Herstellung von Polycarbonat sind als Zweiphasengrenzflächenverfahren grundsätzlich z. B. aus H. Schnell, Chemistry and Physics of Polycarbonates, Polymer Reviews, Vol. 9, Interscience Publishers, New York 1964 S. 33 ff. und auf Polymer Reviews, Vol. 10, "Condensation Polymers by Interfacial and Solution Methods", Paul W. Morgan, Interscience Publishers, New York 1965, Kap. VIII, S. 325 bekannt und die grundlegenden Bedingungen daher dem Fachmann geläufig.

Alternativ können die erfindungsgemäßen Polycarbonate auch nach dem Schmelzumesterungsverfahren hergestellt werden. Das Schmelzumesterungsverfahren ist beispielsweise in der Encyclopedia of Polymer Science, Vol. 10 (1969), Chemistry and Physics of Polycarbonates, Polymer Reviews, H. Schnell, Vol. 9, John Wiley and Sons, Inc. (1964) sowie der DE-C 10 31 512 beschrieben. Beim Schmelzumesterungsverfahren werden die Bisphenole, mit Diarylcarbonaten unter Zuhilfenahme von geeigneten Katalysatoren und gegebenenfalls weiteren Zusatzstoffen in der Schmelze umgeestert.

Auch sind dem Fachmann Verfahren zur Herstellung von Polycarbonaten unter Verwendung der Hydroxyverbindung der Formel (I) bekannt. Beispielsweise kann die Hydroxyverbindung der Formel (I) als Kettenabbrecher in einem Phasengrenzflächenverfahren zur Herstellung von Polycarbonat auf bekannte Art und Weise eingesetzt werden.

Das erfindungsgemäße Verfahren zur Herstellung eines Polycarbonats ist durch die wirtschaftliche Herstellung der Hydroxyverbindung der Formel (I) als eines seiner Edukte ebenso wirtschaftlich vorteilhaft. Ebenso lassen sich durch die Verwendung biobasierter Verbindungen entsprechende biobasierte Polymere herstellen.

In einem weiteren Aspekt der vorliegenden Erfindung wird eine Verwendung mindestens einer Brönsted-Base bereitgestellt zur Erhöhung der Ausbeute einer Hydroxyverbindung der Formel (I) 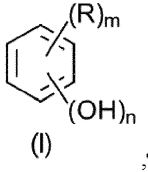 in der
R für eine lineare oder verzweigte Alkylgruppe mit 1 bis 6 Kohlenstoffatomen steht,
n 1 oder 2 ist und
m 0, 1, 2 oder 3 ist,
bei der Reaktion der Decarboxylierung einer Carbonsäureverbindung der Formel (II) oder eines entsprechenden Salzes dieser Carbonsäureverbindung der Formel (II) 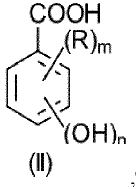
in der R, n und m die oben genannten Bedeutungen haben,
gegebenenfalls unter Verwendung mindestens eines heterogenen Katalysators,
wobei die Brönsted-Base kein Ammoniak, primäres Amin, sekundäres Amin oder ein Gemisch aus zweien oder mehreren dieser stickstoffhaltigen Brönsted-Basen ist und
wobei die Reaktion der Decarboxylierung in einem wässrigen Medium durchgeführt wird.

In dieser erfindungsgemäßen Verwendung gelten die bereits oben in Bezug auf das erfindungsgemäße Verfahren beschriebenen Bevorzugungen und Kombinationen von Bevorzugungen. Insbesondere ist es bevorzugt dass, dass die mindestens eine Brönsted-Base ausgewählt wird aus der Gruppe, bestehend aus Natriumhydroxid, Kaliumhydroxid, Natriumphenolat, Natriumacetat, Natriumphosphat und beliebigen Mischungen daraus.

### Beispiele

### Abkürzungen:

bara: Absolutdruck in bar
U/min: Umdrehungen pro Minute
¹H-NMR: Protonenresonanzspektroskopie
M: Stoffmenegenkonzentration in mol/L
aq.: wässrige Lösung

### Chemikalien:

### 4-Hydroxybenzoesäure (4-HBA): Reinheit ≥ 99 %, Sigma-Aldrich Chemie GmbH

VE-Wasser (H₂O): vollentsalztes Wasser aus Ringleitung
Natriumhydroxid (NaOH): wasserfrei, Reinheit ≥ 97 %, Sigma-Aldrich Chemie GmbH
aq. NaOH-Lösung aus VE-Wasser und Natriumhydroxid hergestellt
Phenol: Reinheit ≥ 96 %, Sigma-Aldrich Chemie GmbH
Hexadeuterodimethylsulfoxid (DMSO-d6): Reinheit ≥ 96 %, Euriso-Top GmbH

### Katalysatoren des Typs Zeolith Y:

Faujasite (Produktreferenz: BCR704).

CBV 600 (CAS 1318-02-1), Zeolyst International, Inc., Oberfläche 660 m²/g, Porengröße 2.43 nm, Si/Al-Verhältnis 2.5. Der Katalysator wurde vor Verwendung 3 h bei 300 °C in Luft kalziniert.

Faujasite, Sigma-Aldrich Chemie GmbH, Oberfläche 567 m²/g, Porengröße 0.67 nm, Si/Al-Verhältnis 1.6. Der Katalysator wurde wie erhalten verwendet.

### Allgemeine Versuchsvorschrift:

0,5 g 4-Hydroxybenzoesäure, 0,5 mL Lösungsmittel (siehe Tabelle 1) und 0,02 g des jeweiligen Katalysators (siehe Tabelle 1) wurden in einem 10 mL-Druckreaktor vorgelegt, mit Argon als Schutzgas gespült und der Reaktor verschlossen. Anschließend wurde ein Argon-Druck von 3 bara beaufschlagt, 10 min bei 800 U/min gerührt und der Druck auf 1,5 bara abgelassen. Dieser Vorgang wurde noch einmal wiederholt, bevor der Reaktor auf die Reaktionstemperatur von 230 °C gebracht wurde. Nach der entsprechenden Reaktionszeit (siehe Tabelle 1) bei dieser Temperatur wurde der Druckreaktor auf Raumtemperatur abgekühlt und der Druck abgelassen. Das erhaltene Reaktionsgemisch wurde in Ethanol aufgenommen, der feste Katalysator wurde mittels Zentrifugation (5 min, 5000 U/min, Hettich Universal 320) abgetrennt und die Lösung am Rotationsverdampfer von Ethanol befreit. Das so isolierte Reaktionsprodukt wurde anschließend mittels ¹H-NMR untersucht.

### ¹H-NMR zur Bestimmung von 4-Hydroxybenzoesäure und Phenol im Reaktionsprodukt:

Etwa 100 mg des gewonnenen Reaktionsprodukts wurden in 0,5 mL DMSO-d6 gelöst und ein ¹H-NMR-Spektrum auf einem Bruker Avance 400 bei 400 MHz vermessen. Die Auswertung der erhaltenen Spektren erfolgte anhand der untenstehenden spezifischen Verschiebungen und Integrale.

| 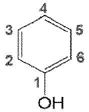 | 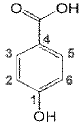 |
|---|---|
| Phenol - ¹H-NMR (400 MHz, DMSO-d6): δ (ppm) 9.4 (C_{OH}, 1H), 7.10-7.20 (C_{3,5}, 2H), 6.72-6.78 (C_{2,4,6}, 3H) | 4-Hydroxybenzoesäure - ¹H-NMR (400 MHz, DMSO-d6): δ (ppm) 12.3 (C_{OOH}, 1H) 10.1 (C_{OH}, 1H), 7.8 (C_{3,5}, 2H), 6.80-6.86 (C_{2,6}, 2H) |

**Tabelle 1:**

| # | Katalysator | Lösungsmittel | Reaktionszeit [h] | Reaktionsprodukt [molares Verhältnis aus ¹H-NMR] |
|---|---|---|---|---|
| 1 | Faujasite | H₂O | 2 | 2:1 |
| | | | | Phenol / 4-Hydroxybenzoesäure |
| 2 | Faujasite | aq. NaOH (0,01 M) | 2 | Phenol |
| 3 | CBV 600 | H₂O | 2 | 2:1 |
| | | | | Phenol/4-Hydroxybenzoesäure |
| 4 | CBV 600 | aq. NaOH (0,01 M) | 2 | Phenol |
| 5 | - | H₂O | 2 | 1:1 |
| | | | | Phenol/4-Hydroxybenzoesäure |
| 6 | - | aq. NaOH (0,01 M) | 2 | Phenol |
| 7 | - | aq. NaOH (0,01 M) | 0,5 | 1:1 |
| | | | | Phenol/4-Hydroxybenzoesäure |

Wie der Tabelle zu entnehmen ist, führt die Zugabe einer Brönsted-Base bei ansonsten gleichen Reaktionsbedingungen zu einer höheren Ausbeute von Phenol (siehe Beispiel 1 (Vergleich) und Beispiel 2 (erfindungsgemäß); Beispiel 3 (Vergleich) und Beispiel 4 (erfindungsgemäß) und Beispiel 5 (Vergleich) und Beispiel 6 (erfindungsgemäß). Dies gilt für unterschiedliche Katalysatoren (Faujasite und CBV 600) und auch ohne die Verwendung eines Katalysators.

Durch den Vergleich der Beispiele 4 (Vergleich), 5 und 6 (erfindungsgemäß) kann zudem beobachtet werden, dass die Reaktion durch die Zugabe der Brönsted-Base beschleunigt wird. Nach einer halben Stunden Reaktionszeit unter Zugabe einer Brönsted-Base (Beispiel 7) erhält man dieselbe Ausbeute wir nach 2 h Reaktionszeit ohne Zugabe einer Brönsted-Base (Beispiel 5).

## Patentansprüche

1. Verfahren zur Herstellung einer Hydroxyverbindung der Formel (I) 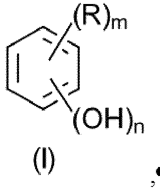 in der
R für eine lineare oder verzweigte Alkylgruppe mit 1 bis 6 Kohlenstoffatomen steht,
n 1 oder 2 ist und
m 0, 1, 2 oder 3 ist,
durch Decarboxylierung einer Carbonsäureverbindung der Formel (II) oder eines entsprechenden Salzes dieser Carbonsäureverbindung der Formel (II) 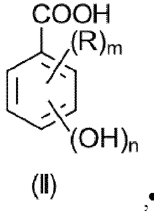 in der R, n und m die oben genannten Bedeutungen haben,
gegebenenfalls unter Verwendung mindestens eines heterogenen Katalysators,
**dadurch gekennzeichnet, dass** bei der Reaktion der Decarboxylierung mindestens eine Brönsted-Base anwesend ist,
wobei die Brönsted-Base kein Ammoniak, primäres Amin, sekundäres Amin oder ein Gemisch aus zweien oder mehreren dieser stickstoffhaltigen Brönsted-Basen ist und
wobei die Reaktion der Decarboxylierung in einem wässrigen Medium durchgeführt wird.

2. Verfahren nach Anspruch 1, **dadurch gekennzeichnet, dass** die mindestens eine Brönsted-Base in einer Konzentration von 0,0001 mol/L bis 20 mol/L, bezogen auf die gesamte Reaktionsmischung der Decarboxylierung, anwesend ist.

3. Verfahren nach Anspruch 1 oder 2, **dadurch gekennzeichnet, dass** die mindestens eine Brönsted-Base ausgewählt wird aus der Gruppe, bestehend aus Natriumhydroxid, Kaliumhydroxid, Natriumphenolat, Natriumacetat, Natriumphosphat und beliebigen Mischungen daraus.

4. Verfahren nach einem der Ansprüche 1 bis 3, **dadurch gekennzeichnet, dass** die Reaktionszeit der Reaktion der Decarboxylierung länger als 5 min und kürzer als 48 h beträgt.

5. Verfahren nach einem der Ansprüche 1 bis 4, **dadurch gekennzeichnet, dass** der gegebenenfalls verwendete mindestens eine heterogene Katalysator ein Zeolith ist.

6. Verfahren nach Anspruch 5, **dadurch gekennzeichnet, dass** das Zeolith eine Faujasit-Struktur aufweist.

7. Verfahren nach einem der Ansprüche 1 bis 6, **dadurch gekennzeichnet, dass** das Kation des Salzes der Carbonsäureverbindung der Formel (II) ausgewählt wird aus der Gruppe bestehend aus Alkalimetallkationen, Erdalkalimetallkationen, Ammonium, Phosphonium, Kationen von Mangan, Eisen, Kobalt, Nickel, Kupfer, Zink, Molybdän, Cadmium und beliebigen Mischungen davon.

8. Verfahren nach einem der Ansprüche 1 bis 7, **dadurch gekennzeichnet, dass** die Carbonsäureverbindung der Formel (II) oder das entsprechende Salz der Carbonsäureverbindung der Formel (II) durch Fermentation oder aus Zuckern, Lignocellulose, lignocellulose-haltigen Materialien, Furanen und/oder Lignin erhalten wurde.

9. Verfahren nach einem der Ansprüche 1 bis 8, **dadurch gekennzeichnet, dass** die Hydroxyverbindung der Formel (I) Phenol ist.

10. Verfahren nach einem der Ansprüche 1 bis 9, **dadurch gekennzeichnet, dass** die Carbonsäureverbindung der Formel (II) oder das entsprechende Salz der Carbonsäureverbindung der Formel (II) ausgewählt wird aus der Gruppe, bestehend aus 2-Hydroxybenzoesäure, 4-Hydroxybenzoesäure und den entsprechenden Salzen.

11. Verfahren zur Herstellung eines Diarylcarbonats oder eines Bisphenols, **dadurch gekennzeichnet, dass** es die folgenden Schritte umfasst:
(i) Durchführung des Verfahrens nach einem der Ansprüche 1 bis 10, um eine Hydroxyverbindung der Formel (I) zu erhalten und
(ii) Reaktion der Hydroxyverbindung der Formel (I) aus Schritt (i) mit mindestens einem Keton, um ein Bisphenol zu erhalten oder Reaktion der Hydroxyverbindung der Formel (I) aus Schritt (i) mit Phosgen, um ein Diarylcarbonat zu erhalten.

12. Verfahren zur Herstellung eines Polycarbonats unter Polymerisation mindestens eines Bisphenols und/oder mindestens eines Diarylcarbonats, **dadurch gekennzeichnet, dass** entweder
(A) das mindestens eine Bisphenol gemäß dem Verfahren nach Anspruch 11 hergestellt wird und/oder das mindestens eine Diarylcarbonat gemäß dem Verfahren nach Anspruch 11 hergestellt wurde oder
(B) dass eine Hydroxyverbindung der Formel (I) hergestellt wird nach dem Verfahren nach einem der Ansprüche 1 bis 10 und anschließend als Kettenabbrecher in der Polymerisation des Polycarbonats nach dem Phasengrenzflächenverfahren eingesetzt wird.

13. Verwendung mindestens einer Brönsted-Base zur Erhöhung der Ausbeute einer Hydroxyverbindung der Formel (I) 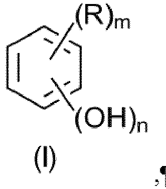 in der
R für eine lineare oder verzweigte Alkylgruppe mit 1 bis 6 Kohlenstoffatomen steht,
n 1 oder 2 ist und
m 0, 1, 2 oder 3 ist,
bei der Reaktion der Decarboxylierung einer Carbonsäureverbindung der Formel (II) oder eines entsprechenden Salzes dieser Carbonsäureverbindung der Formel (II) 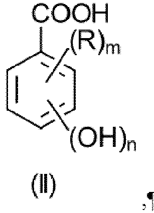
in der R, n und m die oben genannten Bedeutungen haben,
gegebenenfalls unter Verwendung mindestens eines heterogenen Katalysators,
wobei die Brönsted-Base kein Ammoniak, primäres Amin, sekundäres Amin oder ein Gemisch aus zweien oder mehreren dieser stickstoffhaltigen Brönsted-Basen ist und
wobei die Reaktion der Decarboxylierung in einem wässrigen Medium durchgeführt wird.

14. Verwendung nach Anspruch 13, **dadurch gekennzeichnet, dass** die mindestens eine Brönsted-Base ausgewählt wird aus der Gruppe, bestehend aus Natriumhydroxid, Kaliumhydroxid, Natriumphenolat, Natriumacetat, Natriumphosphat und beliebigen Mischungen daraus.

## Claims

1. Process for producing a hydroxy compound of the formula (I) 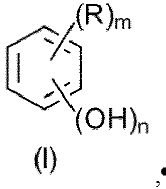 in which
R is a linear or branched alkyl group having 1 to 6 carbon atoms,
n is 1 or 2 and
m is 0, 1, 2, or 3,
by decarboxylation of a carboxylic acid compound of the formula (II) or of a corresponding salt of said carboxylic acid compound of the formula (II) 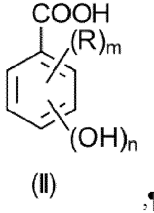
in which R, n and m are as defined above,
optionally with the use of at least one heterogeneous catalyst,
**characterized in that** at least one Brønsted base is present during the decarboxylation reaction,
wherein the Brønsted base is not ammonia, primary amine, secondary amine or a mixture of two or more of said nitrogen-containing Brønsted bases and
wherein the decarboxylation reaction is carried out in an aqueous medium.

2. Process according to Claim 1, **characterized in that** the at least one Brønsted base is present in a concentration of 0.0001 mol/L to 20 mol/L based on the entire decarboxylation reaction mixture.

3. Process according to Claim 1 or 2, **characterized in that** the at least one Brønsted base is selected from the group consisting of sodium hydroxide, potassium hydroxide, sodium phenoxide, sodium acetate, sodium phosphate and any desired mixtures thereof.

4. Process according to any of Claims 1 to 3, **characterized in that** the reaction time of the decarboxylation reaction is longer than 5 min and shorter than 48 h.

5. Process according to any of Claims 1 to 4, **characterized in that** the at least one heterogeneous catalyst optionally used is a zeolite.

6. Process according to Claim 5, **characterized in that** the zeolite has a faujasite structure.

7. Process according to any of Claims 1 to 6, **characterized in that** the cation of the salt of the carboxylic acid compound of the formula (II) is selected from the group consisting of alkali metal cations, alkaline earth metal cations, ammonium, phosphonium, cations of manganese, iron, cobalt, nickel, copper, zinc, molybdenum, cadmium and any desired mixtures thereof.

8. Process according to any of Claims 1 to 7, **characterized in that** the carboxylic acid compound of the formula (II) or corresponding salt of the carboxylic acid compound of the formula (II) was obtained by fermentation or from sugars, lignocellulose, lignocellulose-containing materials, furans and/or lignin.

9. Process according to any of Claims 1 to 8, **characterized in that** the hydroxy compound of the formula (I) is phenol.

10. Process according to any of Claims 1 to 9, **characterized in that** the carboxylic acid compound of the formula (II) or corresponding salt of the carboxylic acid compound of the formula (II) is selected from the group consisting of 2-hydroxybenzoic acid, 4-hydroxybenzoic acid and the corresponding salts.

11. Process for producing a diaryl carbonate or a bisphenol, **characterized in that** the process comprises the following steps:
(i) executing the process according to any of Claims 1 to 10 so as to obtain a hydroxy compound of the formula (I) and
(ii)reacting the hydroxy compound of the formula (I) from step (i) with at least one ketone to obtain a bisphenol or reacting the hydroxy compound of the formula (I) from step (i) with phosgene to obtain a diaryl carbonate.

12. Process for producing a polycarbonate through the polymerization of at least one bisphenol and/or of at least one diaryl carbonate, **characterized in that** either
(A) the at least one bisphenol is produced by the process according to Claim 11 and/or the at least one diaryl carbonate was produced by the process according to Claim 11 or
(B) that a hydroxy compound of the formula (I) is produced by the process according to any of Claims 1 to 10 and is then used as a chain terminator in the polymerization of the polycarbonate by the interfacial process.

13. Use of at least one Brønsted base for increasing the yield of a hydroxy compound of the formula (I) 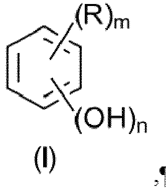 in which
R is a linear or branched alkyl group having 1 to 6 carbon atoms,
n is 1 or 2 and
m is 0, 1, 2, or 3,
in the reaction for the decarboxylation of a carboxylic acid compound of the formula (II) or of a corresponding salt of said carboxylic acid compound of the formula (II) 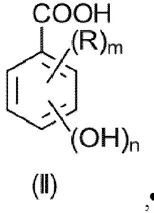
in which R, n and m are as defined above,
optionally with the use of at least one heterogeneous catalyst,
wherein the Brønsted base is not ammonia, primary amine, secondary amine or a mixture of two or more of said nitrogen-containing Brønsted bases and
wherein the decarboxylation reaction is carried out in an aqueous medium.

14. Use according to Claim 13, **characterized in that** the at least one Brønsted base is selected from the group consisting of sodium hydroxide, potassium hydroxide, sodium phenoxide, sodium acetate, sodium phosphate and any desired mixtures thereof.

## Revendications

1. Procédé destiné à préparer un composé hydroxylé de formule (I) 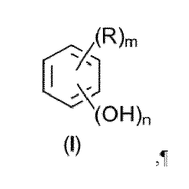 dans laquelle
R représente un groupe alkyle linéaire ou ramifié ayant 1 à 6 atomes de carbone,
n vaut 1 ou 2 et
m vaut 0, 1, 2 ou 3,
par décarboxylation d'un composé acide carboxylique de formule (II) ou d'un sel correspondant de ce composé acide carboxylique de formule (II) 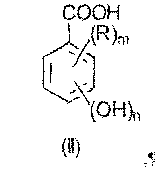
dans laquelle R, n et m ont les significations données ci-dessus,
en utilisant éventuellement au moins un catalyseur hétérogène,
**caractérisé en ce qu'**au moins une base de Brönsted est présente dans la réaction de décarboxylation,
la base de Brönsted n'étant pas de l'ammoniac, une amine primaire, une amine secondaire ou un mélange constitué d'au moins deux de ces bases de Brönsted azotées et
la réaction de décarboxylation étant réalisée dans un milieu aqueux.

2. Procédé selon la revendication 1, **caractérisé en ce que** l'au moins une base de Brönsted est présente à une concentration de 0,0001 mol/l à 20 mol/l, par rapport à la totalité du mélange réactionnel de décarboxylation.

3. Procédé selon la revendication 1 ou 2, **caractérisé en ce que** l'au moins une base de Brönsted est choisie dans le groupe constitué par l'hydroxyde de sodium, l'hydroxyde de potassium, le phénolate de sodium, l'acétate de sodium, phosphate de sodium et de n'importe quels mélanges associés.

4. Procédé selon l'une quelconque des revendications 1 à 3, **caractérisé en ce que** la durée de la réaction de décarboxylation est supérieure à 5 min et inférieure à 48 h.

5. Procédé selon l'une quelconque des revendications 1 à 4, **caractérisé en ce que** l'au moins un catalyseur hétérogène éventuellement utilisé est une zéolithe.

6. Procédé selon la revendication 5, **caractérisé en ce que** la zéolithe présente une structure de type faujasite.

7. Procédé selon la revendication 1 à 6, **caractérisé en ce que** le cation du sel du composé acide carboxylique de formule (II) est choisi dans le groupe constitué par des cations de métaux alcalins, des cations de métaux alcalino-terreux, ammonium, phosphonium, des cations de manganèse, de fer, de cobalt, de nickel, de cuivre, de zinc, de molybdène, de cadmium et de n'importe quels mélanges associés.

8. Procédé selon l'une quelconque des revendications 1 à 7, **caractérisé en ce que** le composé acide carboxylique de formule (II) ou le sel correspondant du composé acide carboxylique de formule (II) est obtenu par fermentation ou à partir de sucres, de lignocellulose, de matières contenant de la lignocellulose, de furanes et/ou de lignine.

9. Procédé selon l'une quelconque des revendications 1 à 8, **caractérisé en ce que** le composé hydroxylé de formule (I) est le phénol.

10. Procédé selon l'une quelconque des revendications 1 à 9, **caractérisé en ce que** le composé acide carboxylique de formule (II) ou le sel correspondant du composé acide carboxylique de formule (II) est choisi dans le groupe constitué par l'acide 2-hydroxybenzoïque, l'acide 4-hydroxybenzoïque et des sels correspondants.

11. Procédé destiné à préparer un carbonate de diaryle ou d'un bisphénol, **caractérisé en ce qu'**il comprend les étapes suivantes consistant à :
(i) réaliser le procédé selon l'une quelconque des revendications 1 à 10 pour obtenir un composé hydroxylé de formule (I) et
(ii) faire réagir le composé hydroxylé de formule (I) de l'étape (i) avec au moins une cétone pour obtenir un bisphénol, ou faire réagir le composé hydroxylé de formule (I) de l'étape (i) avec du phosgène pour obtenir un carbonate de diaryle.

12. Procédé destiné à préparer un polycarbonate par polymérisation d'au moins un bisphénol et/ou d'au moins un carbonate de diaryle, **caractérisé en ce que** soit
(A) l'au moins un bisphénol est préparé selon le procédé selon la revendication 11 et/ou l'au moins un carbonate de diaryle a été produit selon le procédé selon la revendication 11, soit **en ce que**
(B) un composé hydroxylé de formule (I) est préparé selon le procédé selon l'une quelconque des revendications 1 à 10 et est ensuite utilisé comme terminateur de chaîne dans la polymérisation du polycarbonate par le procédé d'interfaces de phases.

13. Utilisation d'au moins une base de Brönsted pour augmenter le rendement d'un composé hydroxylé de formule (I) 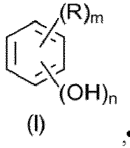 dans laquelle
R représente un groupe alkyle linéaire ou ramifié ayant 1 à 6 atomes de carbone,
n vaut 1 ou 2 et
m vaut 0, 1, 2 ou 3,
lors de la réaction de décarboxylation d'un composé acide carboxylique de formule (II) ou d'un sel correspondant de ce composé acide carboxylique de formule (II) 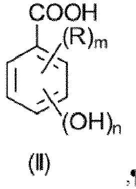
dans laquelle R, n et m ont les significations données ci-dessus,
en utilisant éventuellement au moins un catalyseur hétérogène,
la base de Brönsted n'étant pas de l'ammoniac, une amine primaire, une amine secondaire ou un mélange constitué d'au moins deux de ces bases de Brönsted azotées et
la réaction de décarboxylation étant réalisée dans un milieu aqueux.

14. Utilisation selon la revendication 13, **caractérisée en ce que** l'au moins une base de Brönsted est choisie dans le groupe constitué par l'hydroxyde de sodium, l'hydroxyde de potassium, le phénolate de sodium, l'acétate de sodium, le phosphate de sodium et de n'importe quels mélanges associés.
